# EUROPEAN PATENT SPECIFICATION

(11) **EP 4 112 723 B1**
(45) Date of publication and mention of the grant of the patent: **03.07.2024**
(21) Application number: 21870568.9
(22) Date of filing: 03.12.2021
(51) Int. Cl.: C12N 5/20, C07K 16/40, C12N 15/13, G01N 33/573, G01N 33/577, C07K 16/12

(54) **ANTI-KPC TYPE CARBAPENEMASE HYBRIDOMA CELL LINE, MONOCLONAL ANTIBODY AND APPLICATION THEREOF**
KPC-TYP-CARBAPENEMASE-RESISTENTE HYBRIDOMA-ZELLINIE, MONOKLONALER ANTIKÖRPER (MAB) UND VERWENDUNG DAVON
LIGNÉE CELLULAIRE D'HYBRIDOME DE CARBAPÉNÉMASE DE TYPE ANTI-KPC, ANTICORPS MONOCLONAL ET SON APPLICATION

(30) Priority: 12.05.2021 CN 202110514080
(43) Date of publication of application: 04.01.2023
(73) Proprietor: Tianjin Era Biology Technology Co., Ltd., Tianjin 300457 (CN); BEIJING GOLD MOUNTAINRIVER TECH DEVELOPMENT CO., LTD., Beijing 100081 (CN); BEIHAI SINLON BIOTECH CO., LTD., Beihai, Guangxi 536000 (CN); ERA BIOLOGY (SUZHOU) CO., LTD., Suzhou, Jiangsu 215008 (CN)
(72) Inventor: Wang, Xing, 300480 Tianjin (CN); He, Yongsheng, 300480 Tianjin (CN); Yuan, Qinghua, 300480 Tianjin (CN); Zhou, Yuehui, 300480 Tianjin (CN); Wang, Yufang, 300480 Tianjin (CN); Zang, Danrong, 300480 Tianjin (CN); Wang, Yamiao, 300480 Tianjin (CN); Li, Shilin, 300480 Tianjin (CN)
(74) Representative: Bayramoglu et al.
(86) International application number: PCT/CN2021/135406
(87) International publication number: WO 2022/237138

(56) References cited:
- EP-A1- 3 230 741
- WO-A1-2013/112696
- WO-A1-2013/112696
- WO-A1-2016/092096
- CN-A- 107 667 291
- CN-A- 112 500 489
- CN-A- 112 501 131
- CN-A- 112 980 804
- BOUTAL HERVÉ ET AL: "A multiplex lateral flow immunoassay for the rapid identification of NDM-, KPC-, IMP- and VIM-type and OXA-48-like carbapenemase-producing Enterobacteriaceae", JOURNAL OF ANTIMICROBIAL CHEMOTHERAPY, vol. 73, no. 4, 22 January 2018 (2018-01-22), pages 909-915, XP055841261, GB ISSN: 0305-7453, DOI: 10.1093/jac/dkx521
- ARNOLD RYAN S. ET AL: "Emergence of Klebsiella pneumoniae Carbapenemase-Producing Bacteria :", SOUTHERN MEDICAL JOURNAL, vol. 104, no. 1, 1 January 2011 (2011-01-01), pages 40-45, XP093018906, US ISSN: 0038-4348, DOI: 10.1097/SMJ.0b013e3181fd7d5a Retrieved from the Internet: URL:https://www.ncbi.nlm.nih.gov/pmc/artic les/PMC3075864/pdf/nihms-253051.pdf>

## Description

### TECHNICAL FIELD

The present disclosure belongs to the technical field of antibody preparation, and in particular to a KPC-type carbapenemase-resistant hybridoma cell line, and a monoclonal antibody (mAb) and use thereof.

### BACKGROUND

Carbapenems are one of the most effective drugs for controlling clinical pathogen infections. Carbapenemase-producing organisms (CPOs) and carbapenem-resistant *Enterobacteriaceae* (CRE) have become a global public health issue due to their broad-spectrum drug resistance, and related patients have very limited treatment options. *Enterobacteriaceae* bacteria are important pathogens causing nosocomial infection and community-acquired infection. In recent years, due to the wide application of cephalosporins, a proportion of strains producing extended spectrum beta-lactamases (ESBLs) and AmpC enzymes in *Enterobacteriaceae* has been on the rise significantly, and carbapenem antibacterial drugs are the drugs of choice for treating infections of such strains. However, with the large-scale clinical use of imipenem and meropenem, the number of CRE is also increasing year by year. Clinical studies have shown that CRE strains are resistant to a variety of antibacterial drugs, and thus the choice of clinical treatment drugs is obviously limited. In addition, infected patients are often accompanied by concurrent diseases, which brings great difficulties to clinical infection control and treatment.

There are numerous methods for laboratory detection of carbapenemases. The methods mainly include modified Hodge test, Carba NP test, modified carbapenem inactivation method (mCIM), enzyme inhibitor enhancement test, immunogold labeling test, molecular biotechnology, and the like. The research and development of products for the rapid diagnosis of carbapenemase with independent intellectual property rights is of great significance for early typing of drug-resistant strains, guidance of drug use, and improvement of the medical and health level in China.
Boutal Herve et al., discloses a multiplex lateral flow immunoassay for the rapid identification of NDM-, KPC-, IMP- and VIM- type and OXA-48 like carbapenamase-producing Enterobacteriaceae. Its aim is to detect the presence of the 4 carbapenamase families with a sensitivity of 100% without any false negatives. However, it does not focus on the limit of detection of the presence of the carbapenamase and the minimum titer that can be detected. discloses mAbs binding KPC-carbapenemase and their use for detecting carbapenemase (BOUTAL HERVE; ET AL: "A multiplex lateral flow immunoassay for the rapid identification of NDM-, KPC-, IMP- and VIM-type and OXA-48-like carbapenemase-producing Enterobacteriaceae", JOURNAL OF ANTIMICROBIAL CHEMOTHERAPY, vol. 73, no. 4, 22 January 2018, pages 909-915). EP 3 230 741 A1 discloses mAbs binding KPC-carbapenemase and their use for detecting carbapenemase. WO 2013/112696 A1 discloses pharmaceutical compositions having anti-serine carbapenemase antibodies having antibacterial anti-KPC activity which are capable of killing KPC-expressing strains. It does not disclose any information about the capability of the antibody to detect the antigen nor about the lower limit of detection of the presence of the antigen by using the antibody

### SUMMARY

In order to solve the above technical problems, the present disclosure provides an anti-KPC-type carbapenemase hybridoma cell line, and a mAb and use thereof.

The present disclosure adopts the following technical solutions:
An anti-KPC-type carbapenemase antibody is provided, where the anti-KPC-type carbapenemase antibody is: an antibody 1HG11 including a light chain variable region and a heavy chain variable region, where the light chain variable region includes complementary-determining region 1 (CDR1) shown in SEQ ID NO: 1, CDR2 shown in SEQ ID NO: 2, and CDR3 shown in SEQ ID NO: 3; and the heavy chain variable region includes CDR1 shown in SEQ ID NO: 4, CDR2 shown in SEQ ID NO: 5, and CDR3 shown in SEQ ID NO: 6;
   SEQ ID NO: 1
      KASQNVRSAVA (CDRL1)
   SEQ ID NO: 2
      LASNRHT (CDRL2)
   SEQ ID NO: 3
      LQHWNYPYT (CDRL3)
   SEQ ID NO: 4
      GFNIKDYYMH (CDRH1)
   SEQ ID NO: 5
      WIDPEHGDTEYAPKFQG (CDRH2)
   SEQ ID NO: 6
      YGNFWYFNV (CDRH3)
   or,
an antibody 1HD6 including a light chain variable region and a heavy chain variable region, wherein the light chain variable region includes CDR1 shown in SEQ ID NO: 11, CDR2 shown in SEQ ID NO: 12, and CDR3 shown in SEQ ID NO: 13; and the heavy chain variable region includes CDR1 shown in SEQ ID NO: 14, CDR2 shown in SEQ ID NO: 15, and CDR3 shown in SEQ ID NO: 16.
   SEQ ID NO: 11
      SASSSVSYMY (CDRL1)
   SEQ ID NO: 12
      DTSKLAS (CDRL2)
   SEQ ID NO: 13
      QQWSTNPYT (CDRL3)
   SEQ ID NO: 14
      GYTFIDFEMH (CDRH1)
   SEQ ID NO: 15
      AIHPGRGGSAYNPKFKG (CDRH2)
   SEQ ID NO: 16
      SDYGSSFAY (CDRH3)

In the antibody 1HG11, the light chain variable region has an amino acid sequence shown in SEQ ID NO: 7, and the heavy chain variable region may have an amino acid sequence shown in SEQ ID NO: 9,
SEQ ID NO: 7 
DIVLTQSQKFMSTSVGDRVSITCKASQNVRSAVAWYQQKPGQSPKALIYLASNR
HTGVPDRFAGSGSGTDFTLTISNVQSEDLADYFCLQHWNYPYTFGGGTELEIKRADA
SEQ ID NO: 9 
NSEVQLQQSGAEVVRSGASVKLSCTASGFNIKDYYMHWVKQRPEQGLEWIGWI
DPEHGDTEYAPKFQGKATMTADTSSNTAHLQLSSLTSEDAAVYYCNAYGNFWYFNV
WGAGTTLTVSSAKTTAPSSNGRR;
 or,
   in the antibody 1HD6, the light chain variable region may have an amino acid sequence shown in SEQ ID NO: 17, and the heavy chain variable region may have an amino acid sequence shown in SEQ ID NO: 19,
   SEQ ID NO: 17 
DILMTQSPAVMSASPGEKVTMTCSASSSVSYMYWYQQKSGTSPKRWIYDTSKLA
SGVPTRFSGSGSGTSYSLTITNMETEDAATYYCQQWSTNPYTFGGGTKLEIKRADA
   SEQ ID NO: 19

EVQLQQSGAELVRPGASVKLSCKALGYTFIDFEMHWVKQTPVHGLEWIGAIHPG
RGGSAYNPKFKGKATLTADKSSSTAYMELSSLTSEDSAVYYCIRSDYGSSFAYWGQG
TLVTVSAAKTTPP.

A nucleic acid is provided, including nucleotides encoding the anti-KPC-type carbapenemase antibody described above.

Preferably, a nucleotide sequence of the nucleic acid for encoding the light chain variable region of the antibody 1HG11 may be shown in SEQ ID NO: 8, and a nucleotide sequence of the nucleic acid for encoding the heavy chain variable region of the antibody 1HG11 may be shown in SEQ ID NO: 10,
SEQ ID NO: 8 
GACATTGTGCTCACCCAGTCTCAAAAATTCATGTCCACATCAGTAGGAGACA
GGGTCAGTATCACCTGCAAGGCCAGTCAGAATGTTCGTTCTGCTGTAGCCTGGTA
TCAACAGAAACCAGGGCAGTCTCCTAAAGCACTGATTTACTTGGCATCCAACCGG
CACACTGGAGTCCCTGATCGCTTCGCAGGCAGTGGATCTGGGACAGATTTCACTC
TCACCATTAGTAATGTGCAATCTGAAGACCTGGCAGATTATTTCTGTCTGCAACAT
TGGAATTATCCGTACACGTTCGGAGGGGGGACCGAACTGGAAATAAAACGGGCT
GATGCT
SEQ ID NO: 10 
AATTCAGAGGTTCAGCTGCAGCAGTCTGGGGCAGAGGTTGTGAGGTCAGGGG
CCTCAGTCAAGTTGTCCTGCACAGCTTCTGGCTTCAATATTAAAGACTACTATATG
CACTGGGTGAAACAGAGGCCTGAACAGGGCCTGGAGTGGATTGGATGGATTGAT
CCTGAGCATGGTGATACTGAATATGCCCCGAAGTTCCAGGGCAAGGCCACTATGA
CTGCAGACACATCCTCCAACACAGCCCACCTGCAACTCAGCAGCCTGACATCTGA
GGACGCTGCCGTCTATTACTGTAATGCGTATGGTAACTTCTGGTACTTCAATGTCT
GGGGCGCAGGGACCACGCTCACCGTCTCTTCAGCCAAAACAACAGCCCCATCGTC
GAACGGCAGGCGT;
 or,
a nucleotide sequence of the nucleic acid for encoding the light chain variable region of the antibody 1HD6 may be shown in SEQ ID NO: 18, and a nucleotide sequence of the nucleic acid for encoding the heavy chain variable region of the antibody 1HD6 may be shown in SEQ ID NO: 20,
SEQ ID NO: 18 
GACATTCTGATGACACAGTCTCCAGCAGTCATGTCTGCATCTCCAGGGGAGA
AGGTCACCATGACCTGCAGTGCCAGCTCAAGTGTAAGTTACATGTACTGGTACCA
GCAGAAGTCAGGCACCTCCCCCAAAAGATGGATTTATGACACATCCAAACTGGCT
TCTGGAGTCCCTACTCGCTTCAGTGGCAGTGGGTCTGGGACCTCTTACTCTCTCAC
AATCACCAACATGGAGACTGAGGATGCTGCCACTTATTACTGCCAGCAGTGGAGT
ACTAACCCGTACACATTCGGAGGGGGGACCAAGCTGGAAATAAAACGGGCTGAT
GCTGC
SEQ ID NO: 20

GATGAGGTGCAGCTGCAGCAATCTGGGGCTGAGCTGGTGAGGCCTGGGGCTT
CAGTGAAGCTGTCCTGCAAGGCTTTGGGCTACACATTTATTGACTTTGAAATGCA
CTGGGTGAAGCAGACACCTGTGCATGGCCTGGAATGGATTGGAGCTATTCATCCA
GGAAGGGGTGGTAGTGCCTACAATCCGAAGTTCAAGGGCAAGGCCACACTGACT
GCAGACAAATCCTCCAGCACAGCCTACATGGAGCTCAGCAGCCTGACATCTGAG
GACTCTGCTGTCTATTACTGTATAAGATCCGACTACGGTAGTAGTTTTGCTTACTG
GGGCCAAGGGACTCTGGTCACTGTCTCTGCAGCCAAAACGACACCCCCA.

Use of the anti-KPC-type carbapenemase antibody described above in the preparation of a reagent for detecting a KPC-type carbapenemase antigen is provided.

Preferably, the anti-KPC-type carbapenemase antibody may be used in an *in vitro* diagnostic kit or a microfluidic chip; and the *in vitro* diagnostic kit may be a colloidal gold immunoassay kit, a chemiluminescence kit, a radioimmunoassay kit, an enzyme-linked immunosorbent assay (ELISA) kit, or a fluoroimmunoassay kit.

Preferably, an immune colloidal gold test strip of a double-antibody sandwich method may be prepared, where the antibody 1HG11 is used as a coating antibody and the antibody 1HD6 is used as a gold-labeled antibody; or the antibody 1HD6 is used as a coating antibody and the antibody 1HG11 is used as a gold-labeled antibody.

The present disclosure has the following advantages and positive effects: An anti-KPC-type carbapenemase antibody is provided, and the anti-KPC-type carbapenemase mAb is subjected to systematic evaluation, including evaluation on antibody subtype and titer, and kit sensitivity, specificity, and stability. The anti-KPC-type carbapenemase mAb shows prominent performance in all aspects, and thus is suitable as an immunodiagnostic reagent for *in vitro* diagnosis of KPC carbapenemase, with a titer of more than 1:1,280,000. A prepared kit or microfluidic chip can be used for early typing of drug-resistant strains, guidance of drug use, and assistance of clinical infection control and treatment.

### BRIEF DESCRIPTION OF THE DRAWINGS

FIG. 1 is an electrophoretogram of the KPC-type carbapenemase protein;
FIG. 2 is an electrophoretogram of the anti-KPC-type carbapenemase antibody protein; and
FIG. 3 shows a test result of the KPC-type carbapenemase on a test card of the colloidal gold method.

### DETAILED DESCRIPTION OF THE EMBODIMENTS

The embodiments of the present disclosure will be described below.

The present disclosure relates to a KPC-type carbapenemase-resistant hybridoma cell line named 1HG11.

The present disclosure also relates to a KPC-type carbapenemase-resistant hybridoma cell line named HD6.

The KPC-type carbapenemase-resistant hybridoma cell line capable of stably secreting an anti-KPC-type carbapenemase antibody and a variable region sequence thereof provided by the present disclosure are obtained through mouse hybridoma mAb screening and reverse transcription-polymerase chain reaction (RT-PCR) to clone an Ig variable region gene, and the binding specificity of the antibody is identified by ELISA.

An antibody 1HG11 produced by the KPC-type carbapenemase-resistant hybridoma cell line includes a light chain variable region and a heavy chain variable region, where the light chain variable region includes CDR1 shown in SEQ ID NO: 1, CDR2 shown in SEQ ID NO: 2, and CDR3 shown in SEQ ID NO: 3; and
SEQ ID NO: 1
   KASQNVRSAVA (CDRL1)
SEQ ID NO: 2
   LASNRHT (CDRL2)
SEQ ID NO: 3
   LQHWNYPYT (CDRL3)
the heavy chain variable region includes CDR1 shown in SEQ ID NO: 4, CDR2 shown in SEQ ID NO: 5, and CDR3 shown in SEQ ID NO: 6.
SEQ ID NO: 4
   GFNIKDYYMH (CDRH1)
SEQ ID NO: 5
   WIDPEHGDTEYAPKFQG (CDRH2)
SEQ ID NO: 6
   YGNFWYFNV (CDRH3)

In the antibody 1HG11, the light chain variable region may have an amino acid sequence shown in SEQ ID NO: 7, and the heavy chain variable region may have an amino acid sequence shown in SEQ ID NO: 9;
SEQ ID NO: 7 
DIVLTQSQKFMSTSVGDRVSITCKASQNVRSAVAWYQQKPGQSPKALIYLASNR
HTGVPDRFAGSGSGTDFTLTISNVQSEDLADYFCLQHWNYPYTFGGGTELEIKRADA
SEQ ID NO: 9 
NSEVQLQQSGAEVVRSGASVKLSCTASGFNIKDYYMHWVKQRPEQGLEWIGWI
DPEHGDTEYAPKFQGKATMTADTSSNTAHLQLSSLTSEDAAVYYCNAYGNFWYFNV
WGAGTTLTVSSAKTTAPSSNGRR;
a nucleotide sequence encoding the light chain variable region of the anti-KPC-type carbapenemase antibody may be shown in SEQ ID NO: 8; and
SEQ ID NO: 8 
GACATTGTGCTCACCCAGTCTCAAAAATTCATGTCCACATCAGTAGGAGACA
GGGTCAGTATCACCTGCAAGGCCAGTCAGAATGTTCGTTCTGCTGTAGCCTGGTA
TCAACAGAAACCAGGGCAGTCTCCTAAAGCACTGATTTACTTGGCATCCAACCGG
CACACTGGAGTCCCTGATCGCTTCGCAGGCAGTGGATCTGGGACAGATTTCACTC
TCACCATTAGTAATGTGCAATCTGAAGACCTGGCAGATTATTTCTGTCTGCAACAT
TGGAATTATCCGTACACGTTCGGAGGGGGGACCGAACTGGAAATAAAACGGGCT
GATGCT
a nucleotide sequence encoding the heavy chain variable region of the anti-KPC-type carbapenemase antibody may be shown in SEQ ID NO: 10.
SEQ ID NO: 10

AATTCAGAGGTTCAGCTGCAGCAGTCTGGGGCAGAGGTTGTGAGGTCAGGGG
CCTCAGTCAAGTTGTCCTGCACAGCTTCTGGCTTCAATATTAAAGACTACTATATG
CACTGGGTGAAACAGAGGCCTGAACAGGGCCTGGAGTGGATTGGATGGATTGAT

CCTGAGCATGGTGATACTGAATATGCCCCGAAGTTCCAGGGCAAGGCCACTATGA
CTGCAGACACATCCTCCAACACAGCCCACCTGCAACTCAGCAGCCTGACATCTGA
GGACGCTGCCGTCTATTACTGTAATGCGTATGGTAACTTCTGGTACTTCAATGTCT
GGGGCGCAGGGACCACGCTCACCGTCTCTTCAGCCAAAACAACAGCCCCATCGTC
GAACGGCAGGCGT

An antibody 1HD6 produced by the KPC-type carbapenemase-resistant hybridoma cell line includes a light chain variable region and a heavy chain variable region, where the light chain variable region includes CDR1 shown in SEQ ID NO: 11, CDR2 shown in SEQ ID NO: 12, and CDR3 shown in SEQ ID NO: 13; and
SEQ ID NO: 11
   SASSSVSYMY (CDRL1)
SEQ ID NO: 12
   DTSKLAS (CDRL2)
SEQ ID NO: 13
   QQWSTNPYT (CDRL3)
the heavy chain variable region includes CDR1 shown in SEQ ID NO: 14, CDR2 shown in SEQ ID NO: 15, and CDR3 shown in SEQ ID NO: 16.
SEQ ID NO: 14
   GYTFIDFEMH (CDRH1)
SEQ ID NO: 15
   AIHPGRGGSAYNPKFKG (CDRH2)
SEQ ID NO: 16
   SDYGSSFAY (CDRH3)

In the antibody 1HD6, the light chain variable region may have an amino acid sequence shown in SEQ ID NO: 17, and the heavy chain variable region may have an amino acid sequence shown in SEQ ID NO: 19;
SEQ ID NO: 17 
DILMTQSPAVMSASPGEKVTMTCSASSSVSYMYWYQQKSGTSPKRWIYDTSKLA
SGVPTRFSGSGSGTSYSLTITNMETEDAATYYCQQWSTNPYTFGGGTKLEIKRADA
SEQ ID NO: 19 
EVQLQQSGAELVRPGASVKLSCKALGYTFIDFEMHWVKQTPVHGLEWIGAIHPG
RGGSAYNPKFKGKATLTADKSSSTAYMELSSLTSEDSAVYYCIRSDYGSSFAYWGQG
TLVTVSAAKTTPP
a nucleotide sequence encoding the light chain variable region of the anti-KPC-type carbapenemase antibody may be shown in SEQ ID NO: 18; and
SEQ ID NO: 18 
GACATTCTGATGACACAGTCTCCAGCAGTCATGTCTGCATCTCCAGGGGAGA
AGGTCACCATGACCTGCAGTGCCAGCTCAAGTGTAAGTTACATGTACTGGTACCA
GCAGAAGTCAGGCACCTCCCCCAAAAGATGGATTTATGACACATCCAAACTGGCT
TCTGGAGTCCCTACTCGCTTCAGTGGCAGTGGGTCTGGGACCTCTTACTCTCTCAC
AATCACCAACATGGAGACTGAGGATGCTGCCACTTATTACTGCCAGCAGTGGAGT
ACTAACCCGTACACATTCGGAGGGGGGACCAAGCTGGAAATAAAACGGGCTGAT
GCTGC
a nucleotide sequence encoding the light chain variable region of the anti-KPC-type carbapenemase antibody may be shown in SEQ ID NO: 20; and
SEQ ID NO: 20

GATGAGGTGCAGCTGCAGCAATCTGGGGCTGAGCTGGTGAGGCCTGGGGCTT
CAGTGAAGCTGTCCTGCAAGGCTTTGGGCTACACATTTATTGACTTTGAAATGCA
CTGGGTGAAGCAGACACCTGTGCATGGCCTGGAATGGATTGGAGCTATTCATCCA
GGAAGGGGTGGTAGTGCCTACAATCCGAAGTTCAAGGGCAAGGCCACACTGACT
GCAGACAAATCCTCCAGCACAGCCTACATGGAGCTCAGCAGCCTGACATCTGAG
GACTCTGCTGTCTATTACTGTATAAGATCCGACTACGGTAGTAGTTTTGCTTACTG
GGGCCAAGGGACTCTGGTCACTGTCTCTGCAGCCAAAACGACACCCCCA

The anti-KPC-type carbapenemase antibodies produced by the hybridoma cell line 1HG11 and the hybridoma cell line 1HD6 can be used to detect a KPC-type carbapenemase antigen. The anti-KPC-type carbapenemase mAb shows prominent performance in all aspects, and thus is suitable as an immunodiagnostic reagent for the preparation of an *in vitro* diagnostic kit, and the *in vitro* diagnostic kit may be a colloidal gold immunoassay kit, a chemiluminescence kit, a radioimmunoassay kit, an ELISA kit, or a fluoroimmunoassay kit. Alternatively, the anti-KPC-type carbapenemase antibody can be made into a microfluidic chip for detecting a KPC-type carbapenemase antigen.

To develop a KPC-type carbapenemase diagnostic reagent of the colloidal gold method, a KPC-type carbapenemase antigen first needs to be extracted to obtain a high-purity antigen, the antigen is used to stimulate an excellent immune response in mice, and then the hybridoma technique is used to screen an antibody with high affinity and specificity for the development of related *in vitro* diagnostic reagents. The two antibodies involved in this solution are especially suitable for preparing an immune colloidal gold test strip of a double-antibody sandwich method, where the antibody 1HG11 is used as a coating antibody and the antibody 1HD6 is used as a gold-labeled antibody. The prepared immune colloidal gold test strip of a double-antibody sandwich method is more sensitive. Moreover, the antibody 1HG11 can be used as a gold-labeled antibody, and the antibody 1HD6 can be used as a coating antibody. The present disclosure is further described below through specific examples. Experimental methods for which operation steps are not specified are all conducted in accordance with the corresponding product instructions. The instruments, reagents, and consumables used in the examples can be purchased from commercial companies unless otherwise specified.

### Example 1: Preparation of an anti-KPC-type carbapenemase antibody

### 1.1 Antigen preparation

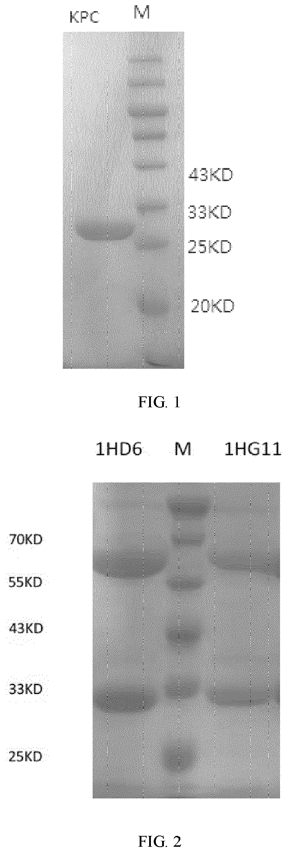

A KPC-type gene sequence was searched and downloaded from National Center for Biotechnology Information (NCBI), subjected to gene synthesis, and ligated to a pet-28a vector, and then the vector plasmid pet-28a-KPC carrying the target gene was transformed into an expression host *Rosseta* (DE3). The host was cultivated until an OD value of a bacterial solution was of 0.4 to 0.6, then isopropylthio-β-galactoside (IPTG) was added at a final concentration of 1 mM for induction, and the host was further cultivated at 37°C for 3 hours. Resulting bacterial cells were collected by centrifugation, then resuspended by adding 30 mL of phosphate buffered saline (PBS) to every about 1 g of the bacterial cells, and then subjected to ultrasonication at a power of 400 W for about 30 min (ultrasonic time: 3 s, interval: 5 s); and after a bacterial solution was non-viscous and clear, the bacterial solution was centrifuged to remove bacterial debris, and a resulting supernatant was filtered through a 0.45 µm filter membrane and loaded on a nickel column packed and equilibrated with an equilibration buffer in advance. After the loading was completed, gradient elution was conducted with imidazole eluents and washing liquids at concentrations of 20 mM, 50 mM, 100 mM, 150 mM, 250 mM, and 500 mM. When the imidazole concentration was 250 mM and 500 mM, a target protein with high purity could be obtained, and a molecular weight thereof was consistent with the expected 29.6 kD. A sodium dodecyl sulphate-polyacrylamide gel electrophoresis (SDS-PAGE) result was shown in FIG. 1. The protein was quantified by bicinchoninic acid (BCA) assay and then dispensed.

### 1.2 Mouse immunization

6-week-old female Balb/c mice were immunized with the purified KPC-type carbapenemase to prepare an antibody, with a protein content of 0.1 mg/ml. The mice were divided into 2 groups according to the immunization dosage, with 5 mice in each group; according to an antigen content, a first group was immunized at an immunization dosage of 25 µg/mouse, and a second group was immunized at an immunization dosage of 50 µg/mouse. For an initial immunization, an appropriate amount of the KPC-type carbapenemase was taken and diluted with distilled water to 300 µL, then 300 µL of complete Freund's adjuvant (CFA) was added for thorough emulsification, and a resulting emulsion was subcutaneously injected into the mice (multi-point injection); two weeks later, the mice were subjected to a secondary immunization by intraperitoneal injection at the same dosage; two weeks later, the mice were subjected to an additional immunization by intraperitoneal injection; 7 days later, blood was collected from the tail of mice, and a serum titer of the mice was determined by ELISA. Specific steps were as follows: An ELISA plate was coated overnight at 4°C with the KPC-type carbapenemase of 0.2 µg/ml at 100 µL/well, spin-dried, and washed 3 times with phosphate-buffered saline with Tween 20 (PBST). 5% skimmed milk powder was added at 200 µL/well to block at 37°C for 2 hours. Blood was collected from the tail of the mice and then centrifuged at 3,000 rpm to obtain serum, and the serum was serially diluted from 1: 1,000 to 1:512,000 with PBS for later use. The plate was spin-dried and then washed 3 times with PBST, a primary antibody diluted with PBS at 1:1,000 was added at 100 µL/well, and the plate was incubated at 37°C for 1 hour. The plate was spin-dried and then washed 3 times with PBST, a goat anti-mouse secondary antibody diluted with PBS at 1:6,000 was added at 100 µL/well, and the plate was incubated at 37°C for 45 min. The plate was spin-dried and then washed 5 times with PBST, 3,3',5,5'-tetramethylbenzidine (TMB) was added at 100 µL/well, and the plate was incubated at 37°C to allow a chromogenic reaction for 10 min; the chromogenic reaction was stopped, and a value was read.

### 1.3 Cell fusion

Three days before fusion, mice were subjected to a booster immunization by intraperitoneal injection at the same dosage as the previous immunization, without an adjuvant. Feeder cells were prepared one day before fusion. A Balb/c mouse 6-8 weeks old was selected, and eyeballs of the mouse were removed to collect blood; then the mouse was sacrificed by cervical dislocation, disinfected in 75% alcohol for 5 min, and fixed on a tray; the abdominal skin was aseptically cut in a clean bench. 10 ml of an HAT selection medium was injected into the abdominal cavity of the mouse with a sterile syringe, the abdomen was slightly rubbed with an alcohol cotton ball, and the medium was drawn out. The medium was added to 40 ml of an HAT medium, and a resulting mixture was added to 4 96-well cell culture plates at 100 µL/well, and then cultivated in a 37°C and 5% CO₂ incubator. One week before fusion, myeloma cells (Sp2/0 cells) were recovered, cultivated in a PRMI-1640 medium with 10% fetal bovine serum (FBS), and subcultivated in a 37°C and 5% CO₂ incubator. Cells in a logarithmic growth phase were collected into a centrifuge tube, subjected to cell counting, and diluted to 10⁷ cells/ml for later use. The Balb/c mice that had been subjected to the booster immunization 3 days prior were taken, eyeballs were removed to collect blood and prepare positive serum, and the mice were sacrificed by cervical dislocation and disinfected with 75% alcohol for 5 min; then a spleen was aseptically collected in a clean bench and washed several times in a sterile dish, and a connective tissue was stripped. The spleen was placed on a microporous copper mesh, a fresh RPMI-1640 medium was added, and the medium was first drawn with a syringe and injected from an end of the spleen to blow off spleen cells; after the process was repeated several times, an inner plug of the syringe was used to gently grind the remaining spleen until there was no obvious red tissue mass. A spleen cell suspension in a dish was gently pipetted up and down, then transferred to a 50 ml centrifuge tube, and centrifuged at 1,000 r/min for 5 min to collect spleen cells, and the spleen cells were counted for later use. The immunized mouse spleen cells and Sp2/0 cells were mixed at a cell number ratio of 10:1, added to a 50 ml centrifuge tube, and centrifuged at 1,000 r/min for 5 min, a resulting supernatant was discarded, and the two cells were thoroughly mixed by rubbing gently in the palm of hands. The centrifuge tube was placed in a 100 mL blue-cap bottle filled with 37°C hot water, and then 1 ml of preheated dimethyl sulfoxide (DMSO)/polyethylene glycol (PEG) was added dropwise to the fusion tube within 1 min, during which the DMSO/PEG was added slowly first and then quickly, and the centrifuge tube was gently swirled. Then an antibiotic-free and serum-free RPMI-1640 medium was immediately added to stop the reaction, during which 1 ml of the medium was added in the first minute, 2 ml of the medium was added in the second minute, 3 ml of the medium was added in the third minute, and 4 ml of the medium was added in the fourth minute. A resulting mixture was incubated in a 37°C water bath for 5 min and then centrifuged at 800 r/min for 5 min, and a resulting supernatant was discarded; a resulting precipitate was suspended with HAT, then thoroughly mixed with 40 ml of an HAT selection medium including 20% calf serum preheated at 37°C, and added to a 96-well cell plate with feeder cells at 100 µL/well, and the cell plate was incubated in a 37°C and 5% CO₂ incubator. 7 days later, half of the medium in the cell plate was replaced with fresh HAT medium, and 10 days later, the medium was completely replaced with HT medium. Positive cells in the 96-well plate were subcloned by limiting dilution analysis (LDA): feeder cells were prepared first according to the above method, and hybridoma cells to be cloned were taken and counted, then diluted to 5 to 8 cells/ml with HT medium, added at 100 µL/well to a 96-well cell plate with the feeder cells (the cloning of each hybridoma cell line required a 96-well cell plate), and cultivated in a 37°C and 5% CO₂ incubator; the number of clones in each cell well was counted and marked about 5 days later, the medium was replaced with fresh medium on day 7, and a test was conducted when cells covered 1/3 to 1/2 of the entire bottom surface of each well; after 2 to 3 times of cloning, all cell wells of the 96-well plate were positive, at which point the cells could be expanded, verified, and cryopreserved. Hybridoma cells verified as positive were expanded and cryopreserved. A specific process was as follows: vigorously-growing hybridoma cells in prominent conditions were gently blown off from a flask with antibiotic-free and serum-free 1640 and then centrifuged at 1,000 r/min for 5 min, and a resulting supernatant was discarded; a cryopreservation solution (with 40% RPMI-1640 medium, 50% FBS, and 10% DMSO) was added to make cells uniformly dispersed, and a resulting cell suspension was then dispensed into cell cryopreservation tubes; then the cryopreservation tubes were placed in a cryopreservation box, the cryopreservation box was placed in a -70°C freezer, and one day later, the cryopreservation tubes were transferred to liquid nitrogen and related records were made.

### 1.4 Ascitic fluid preparation

10 to 12-week-old female Balb/c mice were injected intraperitoneally with sterile liquid paraffin at 0.5 mL/mouse, and 7 days later, the mice were injected intraperitoneally with hybridoma cells in a logarithmic growth phase at 5 × 10⁶ cells/mouse. The mice were observed every day. About 7 to 10 days later, abdomens of the mice obviously bulged, at which point the lower abdominal skin was disinfected with a 75% alcohol cotton ball and then the abdominal cavity was punctured with a 16-gauge needle to collect an ascitic fluid. After an ascitic fluid was regenerated and accumulated, the ascitic fluid was collected once again. The collected ascitic fluid was centrifuged at 3,000 r/min for 10 min, and a resulting clear middle layer was collected, filtered with filter paper, dispensed, and stored at -70°C.

### 1.5 Antibody purification

The ascitic fluid was purified with a Protein-G column. Specific steps were as follows: 2 ml (n) of the ascitic fluid was taken and centrifuged at 10,000 g, and a resulting clear layer was collected and thoroughly mixed with 2 ml of a washing buffer (1:1) to obtain a sample; 20% ethanol was passed through the column, then the column was equilibrated with 8 mL of a washing liquid, and the sample was passed through the column at a flow rate of 8 seconds/drop; the sample was repeatedly loaded 3 times, and then the column was washed with 15 mL of a washing buffer at a flow rate of 8 S/drop; after the washing was completed, elution was conducted with 10 mL of an elution buffer, and after the elution was completed, a pH was adjusted to 7.4 with 1 M Tris PH = 9; then the concentration was conducted with a concentration column, and a resulting concentrate was subjected to dialysis overnight at 4°C with PBS in a 50 kd dialysis bag to obtain an antibody 1HG11 and an antibody 1HD6.

### Example 2: Identification of the anti-KPC-type carbapenemase antibody

### 2.1 Antibody subtype identification

According to the instructions of the SIGMA kit, the mAb subtype identification was conducted by capture-ELISA. Specific steps were as follows: an mAb subtype identification reagent was diluted at 1:1,000, added to an ELISA plate at 100 µL/well, and incubated at 37°C for 1 h; the plate was washed three times with PBST and pat-dried; the antibody was diluted at 1: 1,000 and added at 100 µL/well, and then the plate was incubated at 37°C for 1 h; the plate was washed three times with PBST and pat-dried; an HRP enzyme-labeled goat anti-mouse IgG secondary antibody was diluted at 1:6,000 and added at 100 µL/well, and then the plate was incubated at room temperature for 30 min; a chromogenic reaction was then conducted for 10 min to 20 min. A subtype reagent added in a well with an OD450 reading significantly higher than that of other wells indicated the subtype of the mAb. The antibody subtype of the antibodies 1HG11 and 1HD6 was IgG1.

### 2.2 Antibody titer determination

The antibody titer was determined after purification was conducted by indirect ELISA. Specific steps were as follows: KPC-type carbapenemase was diluted to 0.2 µg/mL and added to an ELISA plate at 100 µL/well to coat overnight at 4°C, where an uncoated control was set; the plate was spin-dried and then washed 3 times with PBST; 5% skimmed milk powder was added at 200 µL/well to block at 37°C for 2 h; the plate was spin-dried and then washed 3 times with PBST; the antibody (with a concentration of 1 mg/ml) was serially diluted from 1: 1,000 to obtain a total of 12 gradient concentrations, resulting antibody dilutions were each added to the plate at 100 µL/well, and the plate was incubated at 37°C for 1 hour, where an uncoated control was set; the plate was spin-dried and then washed 3 times with PBST, a goat anti-mouse secondary antibody diluted with PBS at 1:6,000 was added at 100 µL/well, and the plate was incubated at 37°C for 45 min; the plate was spin-dried and then washed 5 times with PBST, TMB was added at 100 µL/well, and the plate was incubated at 37°C to allow a chromogenic reaction for 10 min; the chromogenic reaction was stopped, and a value was read. After the purification, when the antibody was diluted to 1 mg/ml, a titer reached more than 1: 1,280,000.

### 2.3 Identification of antibody purity and molecular weight

SDS-PAGE was used to identify the molecular weight and purity of the antibody. Gels were prepared, where a separation gel was 12% and a stacking gel was 5%; 20 µL of a sample and 20 µL of a buffer were thoroughly mixed and boiled for 3 min to obtain a sample solution; 20 µL of the sample solution was loaded in each well, and a prestained protein Marker control was set; electrophoresis was conducted at 80 V for 30 min and at 120 V for 2 h; after the electrophoresis was completed, a Coomassie brilliant blue (CBB) solution was added for staining; destaining was conducted by boiling in deionized water 3 times, with 5 min each time; a purified mAb was identified by SDS-PAGE, and resulting bands were clear, without impurity bands. As shown in FIG. 2, there were clear bands at 50 KDa and 25 KDa.

### Example 3: Gene verification of the anti-KPC-type carbapenemase antibody

An Ig variable region gene was cloned by RT-PCR. Total RNA was extracted to synthesize single-stranded cDNA. Total RNA was extracted from the hybridoma cell lines 1HG11 and 1HD6 by the Trizol method (purchased from Invitrogen), and was reverse-transcribed into a cDNA library with M-MLV reverse transcriptase (purchased from Invitrogen).
Upstream primer for a heavy chain framework region
P1: 5'SAGGTGMAGCTKCASSARTCWGG3', as shown in SEQ ID NO: 21
Downstream primer for a heavy chain variable region
P2: 5'TGGGGSTGTYGTTTTGGCTGMRGAGACRGTGA3', as shown in SEQ ID NO: 22
Upstream primer for a light chain leader peptide
P3: 5'ATGGATTTTCAAGTGCAGATTTTCAG3', as shown in SEQ ID NO: 23
Downstream primer for a light chain variable region
P4: 5'GGATACAGTTGGTGCAGCATCAGCCCGTTT3', as shown in SEQ ID NO: 24
A PCR system (50 µL) was prepared as follows:
   cDNA: 2 µL; upstream primer (10 µM): 2 µL; downstream primer (10 µM): 2 µL; dNTP mixture: 2 µL; pfu DNA polymerase (5 U/µL): 1 µL; 10 × pfu Buffer II: 5 µL; and ddH₂O: making up to 50 µL.
   PCR conditions were as follows: pre-denaturation at 95°C for 5 min; repeating the following cycle 35 times: 95°C for 30 s, 58°C for 30 s, and 72°C for 1 min; and finally, extension at 72°C for 10 min.
   VL and VH fragments were separated and recovered by agarose gel electrophoresis. The recovered VL and VH fragments were ligated with a pMD19-T (sKPCle) vector (Takara), and a ligation system was as follows:
      VL PCR product/VH PCR product: each 70 ng; pMD19-T (sKPCle) vector: 1 µL; Solution I ligation reaction solution: 5 µL; and ddH₂O: making up to 10 µL. The ligation was conducted overnight at 4°C.

A ligation product was transformed into *Escherichia coli* (*E. coli*) DH5α competent cells, the competent cells were cultivated overnight at 37°C, single colonies were picked and cultivated at 37°C under shaking for 2 hours, and then a resulting bacterial solution was subjected to PCR identification. cDNA of a corresponding antibody was used as a positive control. A reaction system (25 µL) was prepared as follows:
bacterial solution: 1 µL; upstream primer (10 µM): 1 µL; downstream primer (10 µM): 1 µL; dNTP Mixture (each 2.5 Mm): 2 µL; Taq DNA polymerase (5 U/µL): 0.5 µL; 10 × Taq Buffer (Mg²⁺ plus): 2.5 µL; and water: making up to 25 µL. Reaction conditions were the same as above.

PCR-positive clones were selected for expansion, and a plasmid was extracted from the positive clones with a plasmid extraction kit (Takara) and sequenced. At least 5 clone samples were sequenced for each chain of each antibody until at least three samples had the same sequencing result. The heavy chain and light chain variable region sequences of the antibodies 1HG11 and 1HD6 were successfully cloned, which were consistent with the characteristics of typical antibody variable region sequences according to alignment.

### Example 4: Preparation of a KPC-type carbapenemase detection kit

A KPC-type carbapenemase test card was prepared by a colloidal gold method, and an immune colloidal gold test strip of a double-antibody sandwich method was prepared as follows:
step 1: a 0.1 M K₂CO₃ solution was added to a colloidal gold solution under stirring, a pH was adjusted, and the anti-KPC-type carbapenemase mAb 1HD6 was added; a resulting mixture was stirred, a 10% bovine serum albumin (BSA) solution and 2% PEG 20000 were added, and a resulting mixture was stirred and centrifuged at a low speed to obtain a supernatant; then the supernatant was centrifuged at a high speed to obtain a precipitate, and the precipitate was resuspended with colloidal gold to form a gold-labeled antibody;
step 2: the gold-labeled antibody was sprayed on a sample pad, and then the glass fiber membrane was oven-dried to form a gold-labeled pad;
step 3: a 1% thimerosal sodium solution was thoroughly mixed with the anti-KPC-type carbapenemase mAb 1HG11 to form a test line coating solution; then PBS and a 1% thimerosal sodium solution were added to the goat anti-mouse IgG, and a resulting mixture was thoroughly mixed to form a control line coating solution; the control line coating solution and the test line coating solution were streaked on a nitrocellulose (NC) membrane, and the NC membrane was oven-dried to obtain a coated membrane; and
step 4: the coated membrane was attached to a backing card, the gold-labeled pad and absorbent paper were lapped on the coated membrane, and a resulting product was laminated and cut to obtain the KPC-type carbapenemase test card of the colloidal gold method.

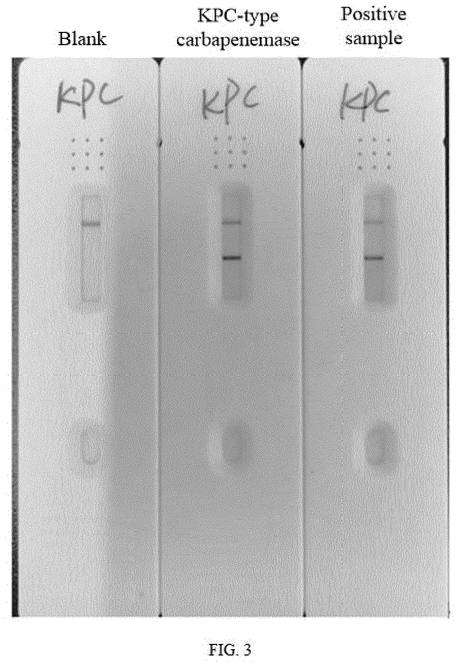

The KPC-type carbapenemase test card of the colloidal gold method prepared by the above method was taken, and a blank sample, KPC-type carbapenemase, and a KPC-type carbapenemase positive sample were each spotted on the test card. Results were shown in FIG. 3, where test results of the blank sample, the KPC-type carbapenemase, and the KPC-type carbapenemase positive sample were shown from left to right, respectively. It can be seen that the test result of the blank sample was negative, and the test results of the KPC-type carbapenemase and the KPC-type carbapenemase positive sample were all positive, indicating that the KPC-type carbapenemase test card of the colloidal gold method prepared by this solution can detect KPC-type carbapenemase and a corresponding positive sample.

The examples of the present disclosure are described above in detail, which are merely preferred examples of the present disclosure and cannot be construed as limiting the scope of implementation of the present disclosure.

## Claims

1. An anti-KPC-type carbapenemase antibody, wherein the anti-KPC-type carbapenemase antibody is: an antibody 1HG11 comprising a light chain variable region and a heavy chain variable region, wherein the light chain variable region comprises complementary-determining region 1 (CDR1) shown in SEQ ID NO: 1, CDR2 shown in SEQ ID NO: 2, and CDR3 shown in SEQ ID NO: 3; and the heavy chain variable region comprises CDR1 shown in SEQ ID NO: 4, CDR2 shown in SEQ ID NO: 5, and CDR3 shown in SEQ ID NO: 6, the light chain variable region has an amino acid sequence shown in SEQ ID NO: 7, and the heavy chain variable region has an amino acid sequence shown in SEQ ID NO: 9;
or,
an antibody 1HD6 comprising a light chain variable region and a heavy chain variable region, wherein the light chain variable region comprises CDR1 shown in SEQ ID NO: 11, CDR2 shown in SEQ ID NO: 12, and CDR3 shown in SEQ ID NO: 13; and the heavy chain variable region comprises CDR1 shown in SEQ ID NO: 14, CDR2 shown in SEQ ID NO: 15, and CDR3 shown in SEQ ID NO: 16, the light chain variable region has an amino acid sequence shown in SEQ ID NO: 17, and the heavy chain variable region has an amino acid sequence shown in SEQ ID NO: 19.

2. A nucleic acid, comprising nucleotides encoding the anti-KPC-type carbapenemase antibody according to claim 1.

3. The nucleic acid according to claim 2, wherein a nucleotide sequence of the nucleic acid for encoding the light chain variable region of the antibody 1HG11 is shown in SEQ ID NO: 8, and a nucleotide sequence of the nucleic acid for encoding the heavy chain variable region of the antibody 1HG11 is shown in SEQ ID NO: 10;
or,
a nucleotide sequence of the nucleic acid for encoding the light chain variable region of the antibody 1HD6 is shown in SEQ ID NO: 18, and a nucleotide sequence of the nucleic acid for encoding the heavy chain variable region of the antibody 1HD6 is shown in SEQ ID NO: 20.

4. Use of the anti-KPC-type carbapenemase antibody according to claim 1 in the preparation of a reagent for detecting a KPC-type carbapenemase antigen.

5. The use of the anti-KPC-type carbapenemase antibody in the preparation of a reagent for detecting a KPC-type carbapenemase antigen according to claim 4, wherein the anti-KPC-type carbapenemase antibody is used in an *in vitro* diagnostic kit or a microfluidic chip; and the *in vitro* diagnostic kit is a colloidal gold immunoassay kit, a chemiluminescence kit, a radioimmunoassay kit, an enzyme-linked immunosorbent assay (ELISA) kit, or a fluoroimmunoassay kit.

6. The use of the anti-KPC-type carbapenemase antibody in the preparation of a reagent for detecting a KPC-type carbapenemase antigen according to claim 4, wherein an immune colloidal gold test strip of a double-antibody sandwich method is prepared; wherein the antibody 1HG11 is used as a coating antibody and the antibody 1HD6 is used as a gold-labeled antibody, or
the antibody 1HD6 is used as a coating antibody and the antibody 1HG11 is used as a gold-labeled antibody.

## Patentansprüche

1. Carbapenemase-Antikörper vom Anti-KPC-Typ, wobei der Carbapenemase-Antikörper vom Anti-KPC-Typ ein Antikörper 1HG11 ist, der eine variable Region der leichten Kette und eine variable Region der schweren Kette umfasst, wobei die variable Region der leichten Kette die komplementärbestimmende Region 1 (CDR1), gezeigt in SEQ ID NO: 1, CDR2, die gezeigt in SEQ ID NO: 2, und CDR3, gezeigt in SEQ ID NO: 3; die variable Region der schweren Kette umfasst CDR1, gezeigt in SEQ ID NO: 4, CDR2, gezeigt in SEQ ID NO: 5, und CDR3, gezeigt in SEQ ID NO: 6, die variable Region der leichten Kette weist eine Aminosäuresequenz auf, die gezeigt ist in SEQ ID NO: 7, und die variable Region der schweren Kette eine Aminosäuresequenz aufweist, die gezeigt ist in SEQ ID NO: 9;
oder
einen Antikörper 1HD6, der eine variable Region der leichten Kette und eine variable Region der schweren Kette umfasst, wobei die variable Region der leichten Kette CDR1, gezeigt in SEQ ID NO: 11, CDR2, gezeigt in SEQ ID NO: 12, und CDR3, gezeigt in SEQ ID NO: 13; die variable Region der schweren Kette umfasst CDR1, gezeigt in SEQ ID NO: 14, CDR2, gezeigt in SEQ ID NO: 15, und CDR3, gezeigt in SEQ ID NO: 16, die variable Region der leichten Kette weist eine Aminosäuresequenz auf, die gezeigt ist in SEQ ID NO: 17 , und die variable Region der schweren Kette eine Aminosäuresequenz aufweist, die gezeigt ist in SEQ ID NO: 19.

2. Nukleinsäure, umfassend Nukleotide, die den Anti-KPC-Typ-Carbapenemase-Antikörper nach Anspruch 1 kodieren.

3. Nukleinsäure gemäß Anspruch 2, wobei eine Nukleotidsequenz der Nukleinsäure zur Kodierung der variablen Region der leichten Kette des Antikörpers 1HG11 in SEQ ID NO: 8 gezeigt ist, und eine Nukleotidsequenz der Nukleinsäure zum Kodieren der variablen Region der schweren Kette des Antikörpers 1HG11,die gezeigt ist in SEQ ID NO: 10;
oder
eine Nukleotidsequenz der Nukleinsäure zum Kodieren der variablen Region der leichten Kette des Antikörpers 1HD6, die gezeigt ist in SEQ ID NO: 18 , und eine Nukleotidsequenz der Nukleinsäure zum Kodieren der variablen Region der schweren Kette des Antikörpers 1HD6, die gezeigt ist in SEQ ID NO: 20.

4. Anwendung des Anti-KPC-Typ-Carbapenemase-Antikörpers nach Anspruch 1 zur Herstellung eines Reagens zum Nachweis eines Carbapenemase-Antigens vom KPC-Typ.

5. Die Anwendung des Anti-KPC-Typ-Carbapenemase-Antikörpers zur Herstellung eines Reagens zum Nachweis eines KPC-Typ-Carbapenemase-Antigens nach Anspruch 4, wobei der Anti-KPC-Typ-Carbapenemase-Antikörper in einem In-vitro-Diagnostik-Kit oder einem mikrofluidischen Chip verwendet wird; und es sich bei dem In-vitro-Diagnostik-Kit um ein Kolloidalgold-Immunoassay-Kit, ein Chemilumineszenz-Kit, ein Radioimmunoassay-Kit, ein Enzym-Linked-Immunosorbent-Assay (ELISA)-Kit oder ein Fluorimmunoassay-Kit handelt.

6. Die Anwendung des Anti-KPC-Typ-Carbapenemase-Antikörpers zur Herstellung eines Reagens zum Nachweis eines KPC-Typ-Carbapenemase-Antigens gemäß Anspruch 4, wobei ein immuner kolloidaler Goldteststreifen eines Doppel-Antikörper-Sandwich-Verfahrens vorbereitet wird, wobei der Antikörper 1HG11 als Beschichtungs-Antikörper und der Antikörper 1HD6 als goldarkierter Antikörper verwendet wird, oder
der Antikörper 1HD6 als Beschichtungs-Antikörper und der Antikörper 1HG11 als goldmarkierter Antikörper verwendet wird.

## Revendications

1. Anticorps anti-carbapénémase de type KPC, dans lequel l'anticorps anti-carbapénémase de type KPC est : un anticorps 1HG11 comprenant une région variable de chaîne légère et une région variable de chaîne lourde, dans lequel la région variable de chaîne légère comprend la région de détermination complémentaire 1 (CDR1) représentée dans SEQ ID NO : 1, CDR2 représentée dans SEQ ID NO : 2, et CDR3 représentée dans SEQ ID NO : 3 ; et la région variable de chaîne lourde comprend la CDR1 représentée dans SEQ ID NO : 4, CDR2 représentée dans SEQ ID NO : 5, et CDR3 représentée dans SEQ ID NO : 6, la région variable de chaîne légère a une séquence d'acides aminés représentée dans SEQ ID NO : 7, et la région variable de chaîne lourde a une séquence d'acides aminés représentée dans SEQ ID NO : 9 ;
ou,
un anticorps 1HD6 comprenant une région variable de chaîne légère et une région variable de chaîne lourde, dans lequel la région variable de chaîne légère comprend CDR1 représentée dans SEQ ID NO : 11, CDR2 représentée dans SEQ ID NO : 12, et CDR3 représentée dans SEQ ID NO : 13 ; et la région variable de chaîne lourde comprend la CDR1 représentée dans SEQ ID NO : 14, CDR2 représentée dans SEQ ID NO : 15, et CDR3 représentée dans SEQ ID NO : 16, la région variable de chaîne légère a une séquence d'acides aminés représentée dans SEQ ID NO : 17, et la région variable de chaîne lourde a une séquence d'acides aminés représentée dans SEQ ID NO : 19.

2. Acide nucléique, comprenant des nucléotides codant pour l'anticorps anti-carbapénémase de type KPC selon la revendication 1.

3. Acide nucléique selon la revendication 2, dans lequel une séquence nucléotidique de l'acide nucléique pour coder la région variable de chaîne légère de l'anticorps 1HG11 est représentée dans SEQ ID NO : 8, et une séquence nucléotidique de l'acide nucléique pour coder la région variable de chaîne lourde de l'anticorps 1HG11 est représentée dans SEQ ID NO : 10 ;
ou,
une séquence nucléotidique de l'acide nucléique pour coder la région variable de chaîne légère de l'anticorps 1HD6 est représentée dans SEQ ID NO : 18, et une séquence nucléotidique de l'acide nucléique pour coder la région variable de chaîne lourde de l'anticorps 1HD6 est représentée dans SEQ ID NO : 20.

4. Utilisation de l'anticorps anti-carbapénémase de type KPC selon la revendication 1 dans la préparation d'un réactif pour détecter un antigène de carbapénémase de type KPC.

5. Utilisation de l'anticorps anti-carbapénémase de type KPC dans la préparation d'un réactif pour détecter un antigène de carbapénémase de type KPC selon la revendication 4, dans lequel l'anticorps anti-carbapénémase de type KPC est utilisé dans un kit de diagnostic in vitro ou une puce microfluidique ; et le kit de diagnostic in vitro est un kit de dosage immunologique d'or colloïdal, un kit de chimioluminescence, un kit de dosage radio-immunologique, un kit de dosage d'immunoabsorption enzymatique (ELISA), ou un kit de dosage immunologique au fluor.

6. Utilisation de l'anticorps anti-carbapénémase de type KPC dans la préparation d'un réactif pour détecter un antigène de carbapénémase de type KPC selon la revendication 4, dans lequel une bande de test d'or colloïdal immunitaire d'un procédé sandwich à double anticorps est préparée, dans lequel l'anticorps 1HG11 est utilisé en tant qu'anticorps de revêtement et l'anticorps 1HD6 est utilisé en tant qu'anticorps marqué à l'or ; ou
l'anticorps 1HD6 est utilisé en tant qu'anticorps de revêtement et l'anticorps 1HG11 est utilisé en tant qu'anticorps marqué à l'or.
